# EUROPÄISCHE PATENTANMELDUNG

(11) **EP 2 821 088 A1**
(43) Veröffentlichungstag der Anmeldung: **07.01.2015**
(21) Anmeldenummer: 13003382.2
(22) Anmeldetag: 03.07.2013
(51) Int. Cl.: A61M 1/00, A61M 1/02, A61B 17/122, A61B 5/15, A61B 17/42

(54) **Halterung für die manuelle Isolierung verschiedener Zelltypen aus der Nabelschnur und ein separates Stativ mit Auflageflächen für das einleitende wie ableitende Schlauch-Spritzen-System**

(71) Anmelder: Haas, Ulrike, 82166 Gräfelfing (DE)
(72) Erfinder: Haas, Ulrike, 82166 Gräfelfing (DE)

(57) **Zusammenfassung**

Die Erfindung beschreibt eine Halterung (Fig. 1) für die manuelle Isolierung verschiedener Zelltypen aus der Nabelschnur und ein separates Stativ mit Auflageflächen für das einleitende, wie ableitende Schlauch-Spritzensystem mit Auffanghalterung (Fig. 3 und 4) der Zellsuspension. Es stellt eine enorme Erleichterung für die manuelle Gewinnung von autologen Zellen für die Transplantation, sowie zur Herstellung von Gewebe zur Transplantation dar. Die Nabelschnur-Halterung ist so aufgebaut, dass zwei Nabelschnurstücke gleichzeitig verarbeitet werden können, um z.B. Endothelzellen aus einer Arterie und einer Vene eines Spenders zu isolieren. Es zeichnet sich im Vergleich zu der bisher üblichen Vorgehensweise dadurch aus, dass die Nabelschnur aufgehängt und justiert wird, wodurch die Hände frei bleiben für andere wichtige Arbeitsschritte.

Somit können aus einer Nabelschnur Endothelzellen und Fibroblasten für Primärkulturen, sowie Progenitorzellen und mesenchymale Stammzellen unkompliziert gewonnen werden.

## Beschreibung

Halterung für die manuelle Isolierung verschiedener Zelltypen aus der Nabelschnur und ein separates Stativ mit Auflageflächen für das einleitende wie ableitende Schlauch-Spritzensystem.

Heute wird insbesondere das Blut der Nabelschnur bei der Geburt eines Kindes, welches bisher ohnehin verworfen wurde, aufgefangen und im tiefgefrorenen Zustand gelagert. Dies wird von vielen Eltern, insbesondere in der Hoffnung durchgeführt, spätere Erkrankungen des Kindes mit Hilfe dieser kryokonservierten Zellen zu therapieren.

Die Erfindung unterstützt daher ebenso das Ziel, Zellen bzw. ein Gewebe, insbesondere für die Eigentransplantation (autologe Transplantation) bereitzustellen, mit welchem das gewünschte Gewebe schnell und sicher und ohne ethische Probleme nach Bedarf bereitgestellt werden kann. Die Erfindung unterstützt außerdem das Ziel, derartiges Gewebe, insbesondere derartiges autologes Gewebe, für Empfänger wie z.B. Kinder bereitzustellen, welches nach der Transplantation mitwächst und daher nicht permanent ausgetauscht werden muss.

Das Arbeiten mit einer Nabelschnur (NS) ist vergleichbar mit dem Halten eines Fisches. Bisher war deshalb die Isolierung von Zellen aus der Nabelschnur eine ziemliche Herausforderung, denn entweder rutschte die ganze Nabelschnur aus der Hand oder die gerade eingesetzte Kanüle rutschte wieder aus dem Blutgefäß usw. Nun soll sozusagen eine Bändigung der Nabelschnur stattfinden, indem sie in einer Halterung, mittels mehrfacher Justierung, senkrecht aufgehängt wird und Schritt für Schritt die einzelnen Reichtümer des Gewebes geerntet werden können. Durch die seitliche Fixierung der Nabelschnur ist ein unbehindertes Arbeiten über die gesamte Länge des Gewebes sichergestellt.

Die Erfindung hat daher zum Ziel, durch die Entwicklung eines preiswerten Gesamtkonzeptes, die Gewinnung unterschiedlicher Zellgruppen aus der Nabelschnur deutlich zu vereinfachen. Bereits nach wenigen Handgriffen, sind die Nabelschnur und die Kanüle an der Nabelschnurhalterung so fixiert, dass erstens die Kanüle während der weiteren Zellisolierung nicht mehr aus dem Gefäß rutschen kann und zweitens die Hände frei sind für die folgenden Arbeitsschritte.

Um aus der Wharton'sche Sulze die Gefäße (2 Arterien, 1 Vene) zu entfernen, ist die Fixierung der NS eine erhebliche Verbesserung der Methode.

Die Fixierung der Nabelschnur ist ebenso ein wichtiger Faktor für das Ausspülen oder Absaugen des NS-Blutes für die spätere Isolierung der Stammzellen. Die Halterung wäre deshalb auch eine Erleichterung für die Hebamme oder Krankenschwester auf der Entbindungsstation, die direkt nach der Geburt das NS-Blut für die Kryokonservierung gewinnen muss.

Zusammenfassend kann das Gesamtkonzept der Nabelschnurhalterung in jedem Forschungslabor, in kommerziellen Unternehmen wie Stammzellbanken oder der Pharmaindustrie und auf den Entbindungsstationen eingesetzt werden.

Abkürzungen:
NS = Nabelschnur

### A. Halterung für die Verarbeitung von Nabelschnüren, d.h. zur Gewinnung von:

Die Nabelschnur besteht aus zwei Arterien, einer Vene und der Wharton'schen Sulze:
   a) in den Blutgefäßen befindet sich noch Blut, das Stammzellen enthält
   b) separate Isolierung von Endothelzellen und Fibroblasten aus der Vene und den Arterien in zwei bis drei Schritten, laut etablierter Vorschrift.
   c) Gewinnung mesenchymaler Stammzellen aus der Wharton'schen Sulze

Zu A. a): NS mit einer Klemme in der Halterung justieren und aus den Gefäßen das Blut absaugen oder die Gefäße mit geeignetem Medium durchspülen (siehe A. b)). Aus dem aufgefangenen Blut können mittels Antikörpern mesenchymale Stammzellen oder Progenitor-Zellen laut etablierter Vorschrift isoliert werden.

### Beispiel zu A. b) Isolierung von Endothelzellen und Fibroblasten

### Vorbereitung der Nabelschnurhalterung:

Einlegen der Metallschale in den Sockel, Anbringen der Klemmen mit Führungsstab (1.5.1) an der inneren Halterung. Kanüle mit Dreiwegehahn am Luer-Lock-Ende anschrauben.

### Durchführung:

Die äußere Haut eines NS-Stückes mit der vorbereiteten Klemme (1.5.1) greifen und daran die NS an der inneren Halterung (1.4.4 - 2. von oben) aufhängen. Einen chirurgischen Faden um das Blutgefäß legen; Kanüle mit Dreiwegehahn in die Vene oder Arterie einsetzen, evtl. das Umfeld des Blutgefäßes mit Skalpell einschneiden. Um ein Abrutschen der Kanüle zu verhindern, wird der Dreiwegehahn in der bereits vorbereiteten Klemme (an 1.4.4 - 1. von oben) justiert. Sobald das Blutgefäß kanüliert ist, wird die Schlinge (z.B. chirurgischer Faden) um das Blutgefäß gelegt und durch einen dreifachen Knoten stramm festgezogen. Mit einer großen Klemme das Gefäß im Bereich der Kanüle umfassen und mit der Flügelschraube in der Gewindebohrung (1.4.7) festschrauben. Falls nötig kann ein weiteres Abdichten rund um das Blutgefäß stattfinden, z.B. mit zwei kleinen Krokodilklemmen. Durch das Abdichten rund um das Blutgefäß, können die eingespritzten Lösungen samt abgelöster Zellen, nicht an der Kanüle vorbei nach außen austreten. Am Dreiwegehahn das Einmalschlauchsystem anschrauben, ebenso am Dreiwegehahn der Spritze, die in der Auflagefläche von 4.1 oder 4.2 ruht. Spüllösung durch das Schlauchsystem und Gefäß leiten, um das restliche Blut zu entfernen. Jetzt findet die Kanülierung des Blutgefäßes am unteren Ende statt. Die Fixierung an der inneren Halterung findet nur am Dreiwegehahn statt. Nachdem die Arterie oder Vene kanüliert wurden, werden über die Spritzen die Spüllösung, die Kollagenase / Protease oder das Trypsin und letztlich die Stopplösung durch ein Schlauchsystem in die Nabelschnur geleitet. Hierbei kann das Blutgefäß durch die Dreiwegehähne beidseitig verschlossen und auch mit der jeweiligen Lösung aufgebläht werden. Je nach Arbeitsschritt verweilt die Lösung längere Zeit in den Gefäßen, um die Zellen herauszulösen.

Die Ableitung der Lösungen aus den Nabelschnur-Blutgefäßen erfolgt über die am unteren Ende der NS angebrachten Kanülen, die ebenso mit Dreiwegehähnen und einem Einmalschlauchsystem verbunden sind, um die durchgelaufenen Flüssigkeiten in Auffangröhrchen oder einem Abfallgefäß aufzufangen. Ebenso ist es möglich, am unteren Ende der NS keine Kanüle anzubringen und das Auffanggefäß direkt unter die NS zu stellen, um so die unterschiedlichen Lösungen aufzufangen. In diesem Fall wird zur Inkubation mit Kollagenase / Protease oder ähnlichem, die Nabelschnur mit einer chirurgischen oder Bulldog-Klemme verschlossen.

Die Inkubation kann im Brutschrank bei 37°C, bzw. auf einer Wärmeplatte unter der Laminar Flow stattfinden. Hierzu kann die innere Halterung (1.4) aus der äußeren Halterung (1. 1) entnommen werden, indem der Metallriegel (1.1.10) nach außen gedreht wird, oder die Halbschale (1.1.11) in deren Führungsschienen nach oben geschoben wird, oder indem die Zapfen (1.1.12) am gemeinsamen Drehpunkt entnommen werden. Die entnommene innere Halterung, samt kanülierter NS, wird dann in ein entsprechend großes Gefäß mit vorgewärmtem Medium gelegt. Die einfachere Lösung ist die Verwendung einer Wärmegelkompresse, die um die Nabelschnur gelegt wird. In diesem Fall sollte evtl. zuvor zur Befeuchtung der NS eine sterile, angefeuchtete Mullkompresse aufgelegt werden.

Die aufgefangene Zellsuspension wird bei 500 g für 7 min zentrifugiert, der Überstand verworfen und das Zellpellet mit 7 ml frischem Medium in eine T 25 Zellkulturflasche überführt. Weitere Kultivierung der Zellen in T175 Zellkulturflaschen für direkte Beschichtung z. B. einer Herzklappe, bzw. Einfrieren der Zellen in Stickstoff für einen späteren Bedarf der Zellen.

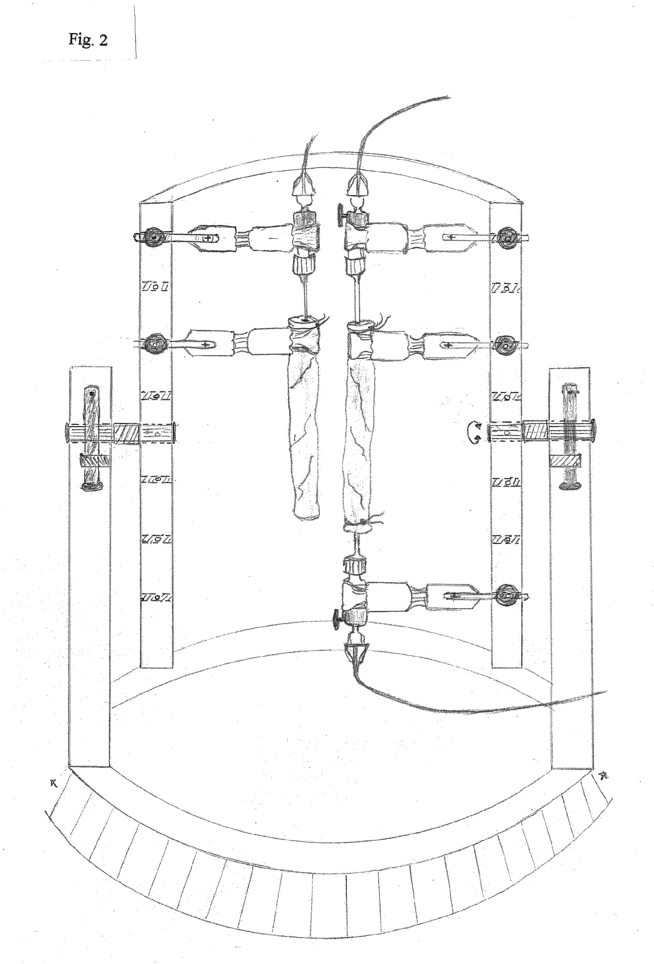

Mit der Aufteilung der NS-Halterung ist es möglich gleichzeitig Endothelzellen bzw. Fibroblasten aus der Vene und einer Arterie der Nabelschnur zu isolieren. Es ist ausreichend die Nabelschnur nur von einer Seite zu arretieren, so daß zwei Nabelschnurstücke nebeneinander in der Halterung angebracht werden können: z. B. auf der linken Seite die Arterie und auf der rechten Seite die Vene verarbeitet wird (Fig. 2 u. 3).

Zu A. c) Es empfiehlt sich die NS in ca. 8 - 10 cm große Abschnitte zu teilen. Zuerst müssen die Gefäße der NS herauspräpariert werden. Dies geschieht, indem die mit einer Klemme in der Halterung justierte NS mit einem Skalpell längs des Gefäßes vorsichtig aufgeschnitten wird und die Gefäße mit einer Pinzette herausgezogen werden. Anschließend erfolgt das Ausschälen und Zerkleinern der Wharton'schen Sulze und ein enzymatischer Verdau des Gewebes mit z. B. Kollagenase, um die mesenchymalen Stammzellen freizulegen. Hierzu gibt es Vorschriften der etablierten Methoden.

### Aufbau der Nabelschnurhalterung:

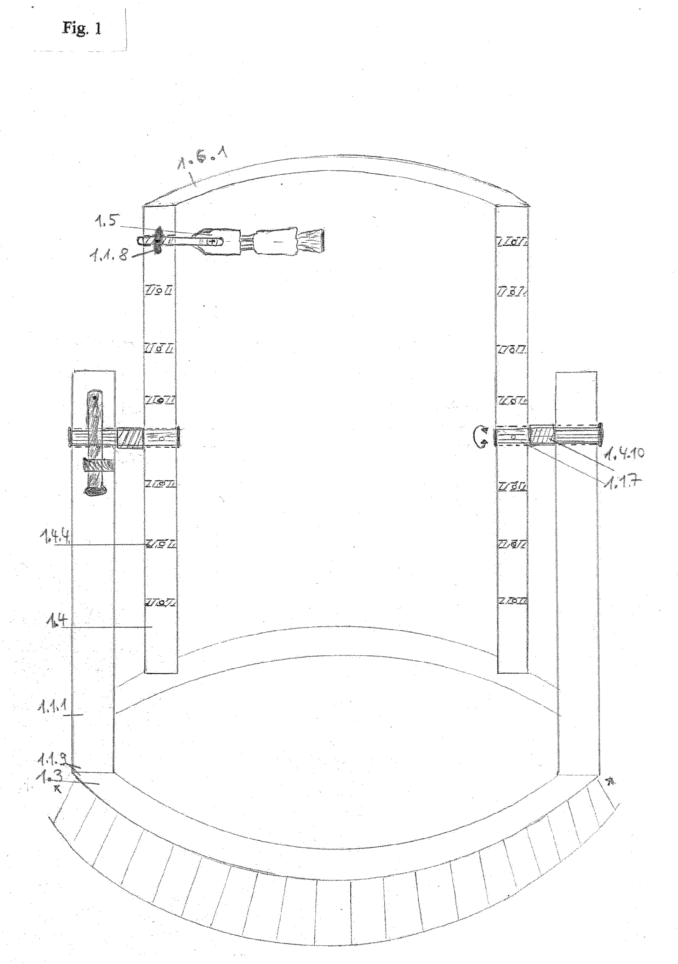

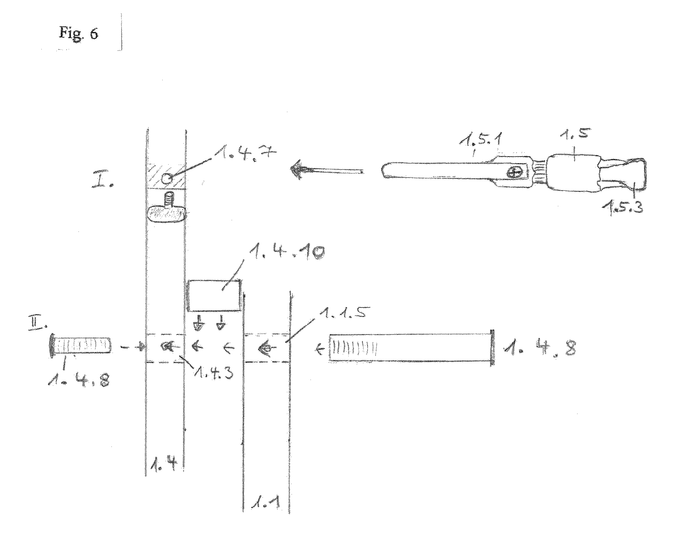

Halterung zur Justierung einer Nabelschnur, bestehend aus Metall oder Kunststoff (Fig. 1)
- äußeres Gestell, bestehend aus zwei senkrechten Stäben (ca. 17 -25 cm hoch), die unten an einem runden oder quadratischen Standsockel (1.2) aus Kunststoff verankert sind, oder einem (z.B. 1,5 x 1,5 cm) Metallkreis der wiederum in einem breiten Metallkranz (1.3) steckt, der nach außen abgeschrägt ist, um die Standfestigkeit zu gewährleisten. Bei Bedarf kann der Metallkreis im Metallkranz gedreht werden.
- innere Halterung die mit (1.1) in Höhe von ca. 15 - 17 cm über einen Zapfen (1.4.8) ohne Begrenzung schwenkbar ist oder durch eine Rasterschraube (1.4.9) verbunden ist und dadurch ca. 70 - 90° nach vorne bzw. 110° nach hinten schwenkbar wird. Der Zapfen läuft zwischen 1.1 und 1.4 durch eine Teflonröhre (1.4.10), deren Länge der, entsprechend des gewählten Zwischenraumes (von 1.1 zu 1.4) entspricht. Die innere Halterung, samt angebrachter Nabelschnur, kann bei Bedarf (1.4.6 und Fig. 6, II), zur Inkubation im Brutschrank entnommen werden: durch Herausheben nach vorne oder Herausziehen der Zapfen, die in dem Fall mit einer Schraube in sich befestigt sind.
- äußere und innere Halterung (1.1. und 1.4) haben einen inneren Abstand von 1,5 - 2 cm Freiraum
- Die inneren Stäbe (1.4) sollten ca. 13 cm Zwischenraum haben, um Bewegungsfreiheit beim Arbeiten zu gewährleisten
- die innere Halterung (1.4) (kleine Variante: H ca. 130 mm; große Variante: H ca. 24 cm) hat oberhalb und unterhalb des mittigen Drehpunktes mehrere Bohrungen, um verschiedene Hilfsmittel anzubringen. Statt der Bohrungen ist zudem ein stufenloses Festklemmen, Verschrauben der verschiedenen Hilfsmittel denkbar.
- an der inneren Halterung (1.4) werden von links und rechts Klemmen (1.5) angebracht, um die NS zu befestigen.
   I. Die Klemmen können wie eine chirurgische Klemme gearbeitet sein, mit Rillen an beiden Innenseiten. Die Klemmenfläche (ca. 1 cm) sollte jedoch senkrecht zur Nabelschnur stehen, oder
   II. die Klemmfläche besteht aus drei kleinen stumpfen Greifkrallen.
   III. Zum Umfassen der NS bzw. der Dreiwegehähne eignen sich Krokodilklemmen oder Bulldog-Klemmen in verschiedenen Größen
- Die Klemmkraft von 1.5.4 wird entweder durch
   I. Einrasten einer Nase in eine Rille gewährleistet
   II. eine starke Metallfeder oder
   III. das Zusammendrücken (öffnen der Klemme), lösen der Zangenblätter (schließen der Klemme)
- die Klemmen sind an Führungsstäbe (1.5.1) (L: insgesamt ca. 70 mm - 11 mm) angeschraubt oder fest verbunden und werden in Bohrungen (1.4.4) an der inneren Halterung (1.4) eingeschoben und z. B. mit einer Flügelschraube (1.5.2) oder flachem bzw. kugelförmigem Schraubkopf in einem Schraubgewinde (1.4.7) an der Frontseite von (1.4) befestigt. (Fig.1 u.6.I)
- die Klemmen (1.5) besitzen für den Vorgang (1.4.7) am äußeren Ende zwei bis drei Rillen (L: 20 - 30 mm), in die die Flügelschraube (1.5.2) eingedreht werden kann, um ein Umkippen der Klemme zu verhindern.
- die innere Halterung (1.4)) hat oben an der rückwärtigen Seite einen Halbring aus Metall oder Kunststoff, der die inneren Stäbe (b) in paralleler Stellung hält und an dem zusätzlich Hilfsmaterial befestigt werden können. Der Halbring ist in 1.4 aufgesteckt oder aufgeschraubt, er kann durch einen kleineren Halbring (Ø 100 mm) ausgewechselt werden. Soll nur ein Blutgefäß kanüliert werden, kann auf den Halbring verzichtet werden, wodurch die Inkubation bei 37°C in einem kleineren Behälter stattfinden kann.
- Die Kanüle ist an einen Dreiwegehahn angeschraubt und wird mit z.B. einer Krokodilklemme (in 1.5.1)) umfaßt, um sie in (1.4.4.) stabil zu arretieren. Dadurch wird das Abrutschen der Kanüle aus dem Blutgefäß verhindert.
- Das äußere Gestell (1.1) hat am Boden eine herausnehmbare Metallschale, um Abfall aufzufangen.
- Am äußeren Gestell ist eine einschwenkbare Lupe angebracht, um die Kanüle besser in das Blutgefäß einführen zu können. Die Lupe kann vor der Sterilisation abgenommen werden. Oder es kann im Bedarfsfall eine separate Lupe dazugestellt werden.

### B. Stativ oder Tisch mit einer Auflagefläche für 3 Spritzen

Jede Spritze (zu A. b)) ist über zwei Dreiwegehähne und einen Schlauch mit der Kanüle verbunden, die in eines der drei Blutgefäße eingeführt wurde. Das Einmalschlauchsystem und der Dreiwegehahn besitzen an einem Ende Luer-Lock-Anschlüsse. Die Metall- oder Kunststoff-Einmalkanüle besitzt ebenfalls einen Luer-Lock-Anschluss.

### Beschreibung der Spritzenauflage:

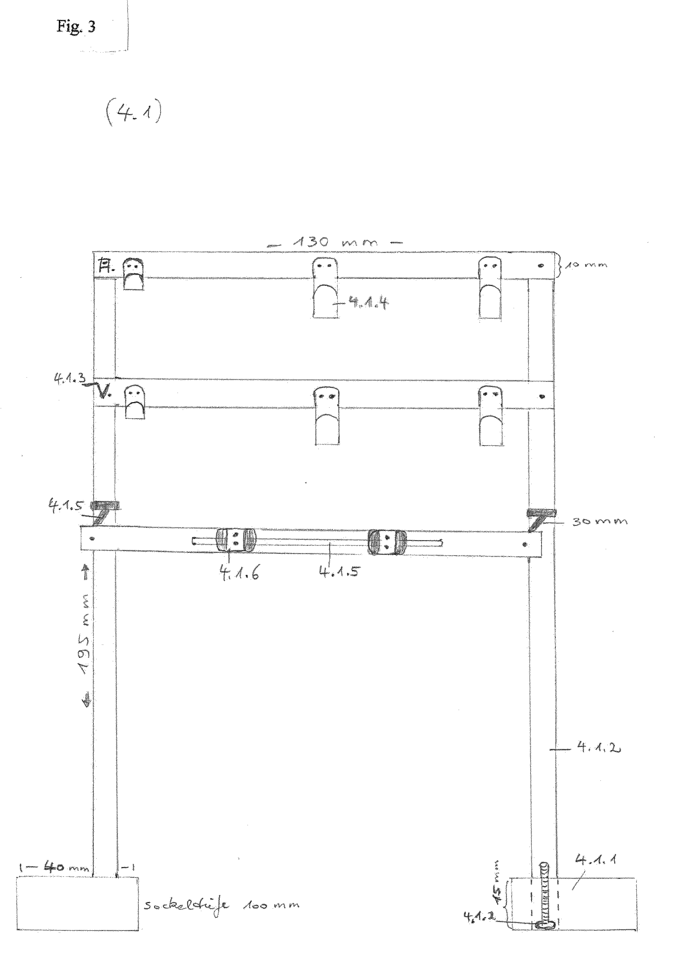

a) ein Stativ, das aus einem Sockel und zwei Metallstäben besteht und das in zwei Etagen, als Querverbindung zwischen den beiden Metallstäben wiederum zwei Metallschienen (A und V) aufweist, diese können auch in einem schrägen Winkel nach unten angebracht sein. Jede dieser Metallschienen besitzt eine kleine offene Metallspange (0 10 mm) für die Dreiwegehähne und jeweils zwei größere offene Metallspangen oder Ringe (Ø 25 mm), in die die 20 ml Spritzen eingeklemmt oder eingeschoben werden können. Die Stempel der Spritzen können außerhalb des Gestells frei bewegt werden (Fig. 3).

Eine dritte Schiene, die durch zwei Stege ca. 30 mm nach vorne versetzt ist, unterhalb von A und V, besitzt zwei kleine, senkrecht stehende, offene Metallspangen (0 10 mm) oder geschlossene Ringe für die Dreiwegehähne des ableitenden Schlauchsystems. Die Metallspangen sind nach links und rechts verschiebbar, damit sie mittig über dem Auffangröhrchen justiert werden können. Die Auffangröhrchen stehen in einem separaten Sockel oder handelsüblichen Röhrchenständer, der unter die Halterung der ableitenden Schläuche mit Dreiwegehähnen gestellt wird (Fig. 3).

Siehe Figur 3: Ein Gestell, das aus einem Sockel (4.1.1) (H: 15 mm; B: 40 mm; L:100 mm) und zwei Metall- oder Kunststoffstangen (4.1.2) (rund oder eckig, H: 195 mm; B; 8 - 15 mm) besteht, die von der Sockelunterseite mit einer Schraube oder mit Gewinde angeschraubt sind. An zwei Querstreben (4.1.3) (L:130 mm; H: 10 mm; B:2 - 3 mm) sind je eine kleine offene Metall- oder Gummispange (Ø 10 mm) und je zwei offene Spangen (4.1.4), es können auch geschlossene Ringe sein, mit einem Durchmesser von 23 - 25 mm angebracht. An einer dritten Schiene (4.1.5) darunter, sitzen zwei Metall- oder Gummispangen (4.1.6) (0 10 mm) frei verschiebbar. Diese Schiene besitzt mittig einen Schlitz und ist durch zwei ca. 30 mm lange Stäbe (4.1.5) nach außen versetzt, um ein freies Arbeiten zu gewährleisten.

Eine andere Möglichkeit ist
b) ein Tisch: Die Auflagefläche für die Spritzen kann an vier Stäben angebracht werden, die auf einem oder zwei soliden Sockeln ruhen.

Die Dreiwegehähne werden an der Auflagefläche in einer offenen Spange oder einer Gummihalterung festgeklemmt. Die mit dem Dreiwegehahn verbundenen Spritzen werden in die Vertiefung der Auflagefläche eingelegt, wodurch ein Verrutschen verhindert wird. Die Stempel der Spritzen können außerhalb der Auflagefläche frei bewegt werden.

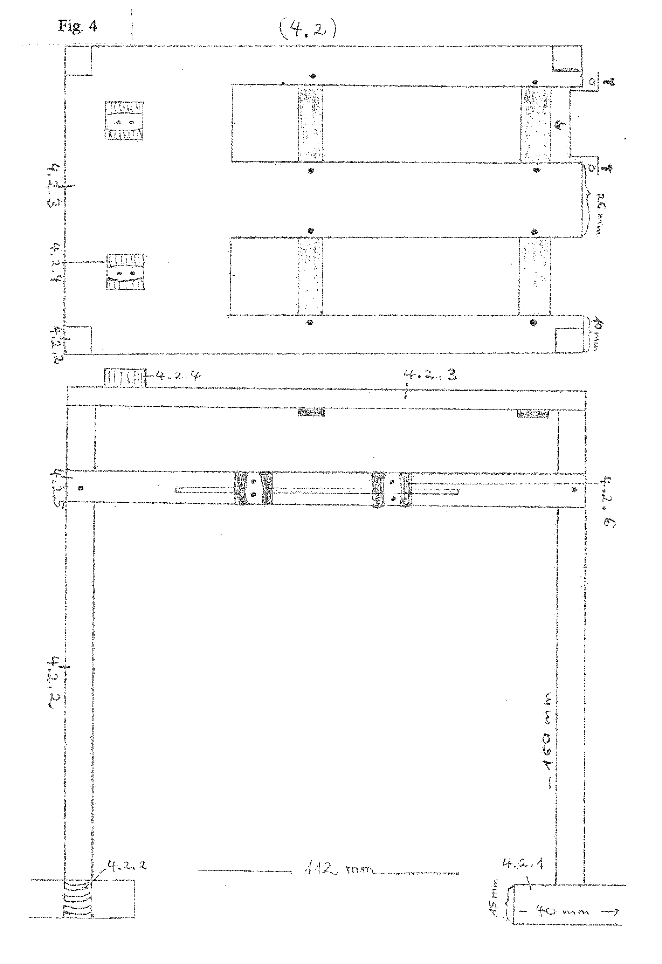

An der Längsseite des Tisches befindet sich unterhalb der Spritzen-Auflagefläche eine Schiene mit zwei kleinen offenen, senkrecht stehenden, Metallspangen (Ø 1 cm) für die Dreiwegehähne des ableitenden Schlauchsystems. Die Metallspangen sind nach links und rechts verschiebbar, damit sie mittig über dem Auffangröhrchen justiert werden können (Fig. 4).

Siehe Fig. 4: 4 Säulen aus Kunststoff oder Metall(H:160 mm; Ø 8 - 12 mm), die von der Sockelunterseite mit einer Schraube oder mit Gewinde (4.2.2) auf dem Kunststoffsockel (4.2.1) (B: 40 mm; H: 5 - 15 mm; L: 110 mm) angeschraubt sind. Auf den Säulen ist die Auflagefläche (4.2.3) aus Kunststoff oder Metall befestigt, die 2 bis 3 Vertiefungen für 20 ml Spritzen aufweist. Die Vertiefung kann durch zwei bis drei U-förmige Stege, H: 8 mm; B: 8 - 20 mm, gebildet werden, die an der Plattform oben oder von unten angeschraubt, angeschweißt sind und die den Spritzen als Auflagefläche dienen. Vor der Vertiefung sind offene Metall- (4.2.4) (Ø 1 cm) oder Gummispangen angebracht, um die Dreiwegehähne mit den Spritzen festzuklemmen. Die Spritzenkolben sind frei beweglich, da die Vertiefung der Auflagefläche an diesem Ende offen ist. Eine Querstrebe ist an der Längsseite von 4.2 angebracht, Diese Schiene besitzt mittig einen Schlitz, in ihm sitzen frei verschiebbar (4.2.5) zwei Metall- oder Gummispangen (Ø 10 mm) (4.2.6), die mit einer Flügelschraube von der Rückseite der Schiene arretiert werden können.

### Bezugszeichenliste:

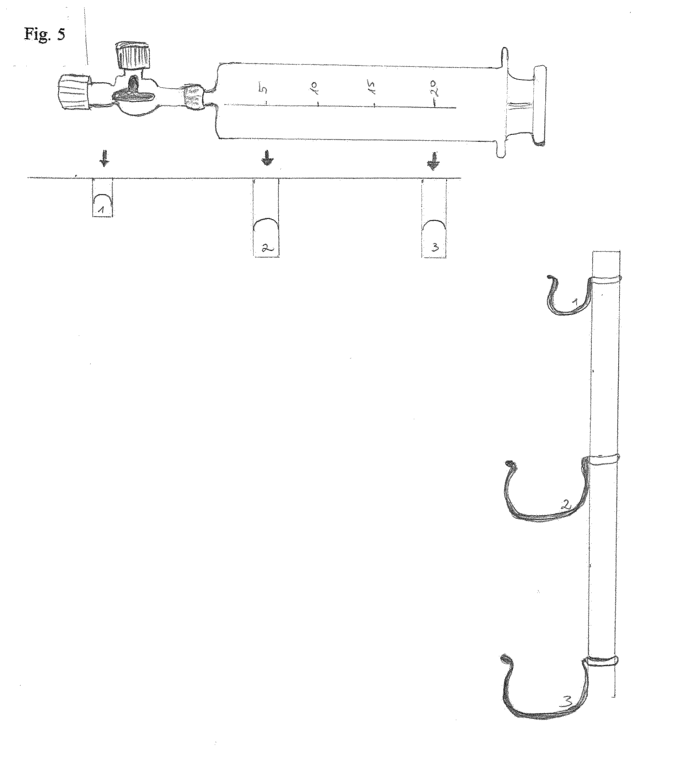

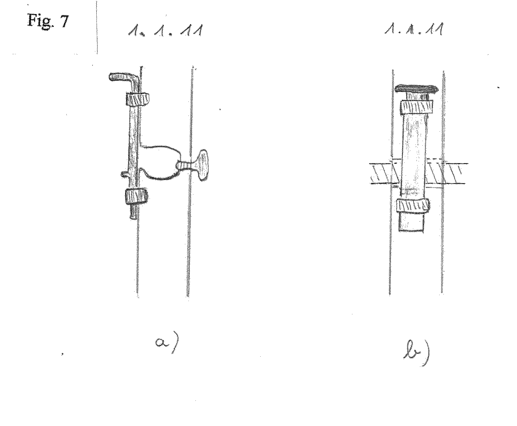

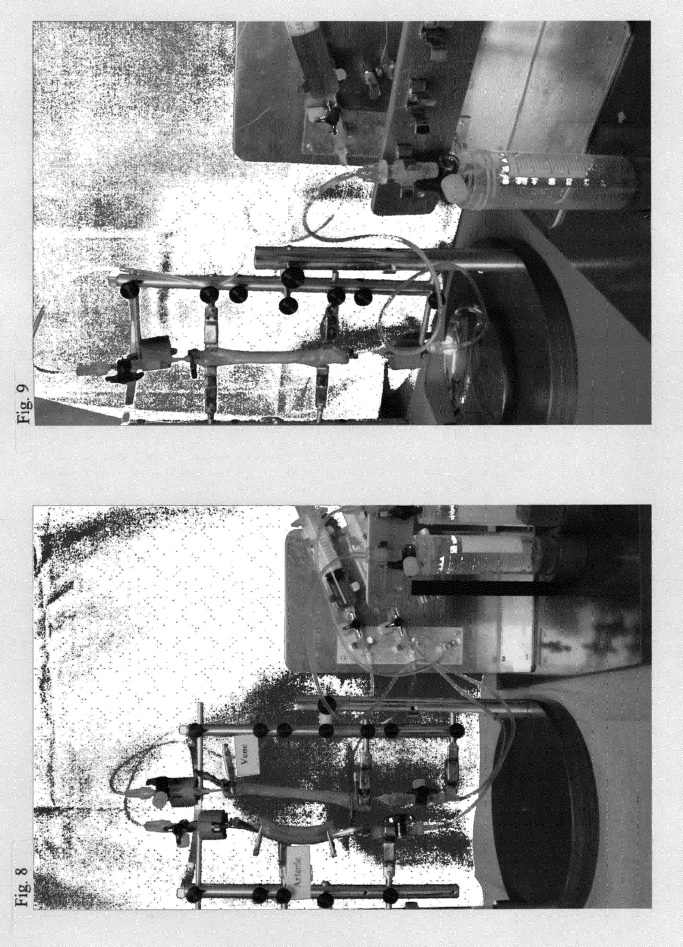

- Fig. 1:: Nabelschnurhalterung
- Fig. 2:: Nabelschnurhalterung mit 2 fixierten, kanülierten (Arterie und Vene) Nabelschnurabschnitten
- Fig. 3:: Halterung für 3 Spritzen mit Querverstrebung und Spangen
- Fig. 4:: Halterung für 2 Spritzen mit Auflagefläche und Befestigung für das Ableitsystem
I. Aufsicht der Auflagefläche mit U-förmigem Einsatz
II. Frontalansicht mit Spangen für Dreiwegehähne, es sind deshalb nur zwei Säulen sichtbar
- Fig. 5:: Teildarstellung der Spritzenaufhängung
- Fig. 6:: I.Justierung der Klemmen mit Flügelschraube in 1.4.7
II. Montageansicht von 1.1 und 1.4 am Drehpunkt 1.1.5
- Fig. 7:: eine Halbschale (1.1.11), um die innere Halterung (1.4) entnehmen zu können:
a) Seitenansicht
b) Frontansicht
- Fig. 8:: Einfache Anfertigung einer Nabelschnurhalterung plus Spritzenauflage: kanülierte Arterie und Vene
- Fig. 9:: Foto einer Nabelschnurhalterung mit eingespannter, kanülierter Nabelschnur und Spritzenhalterung (Stativ) und Auffangröhrchen

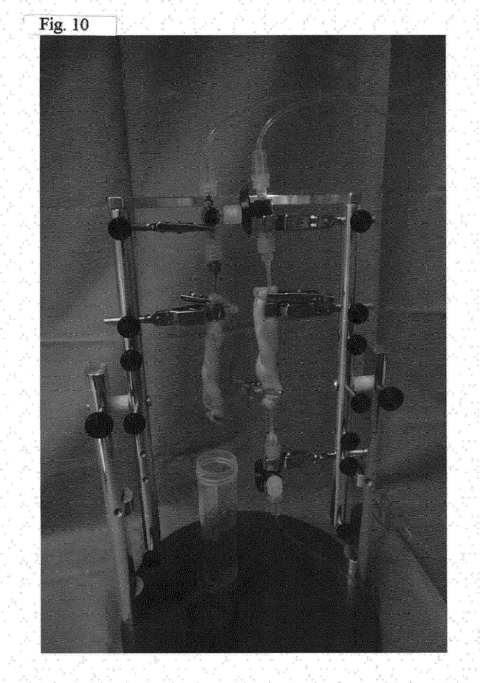

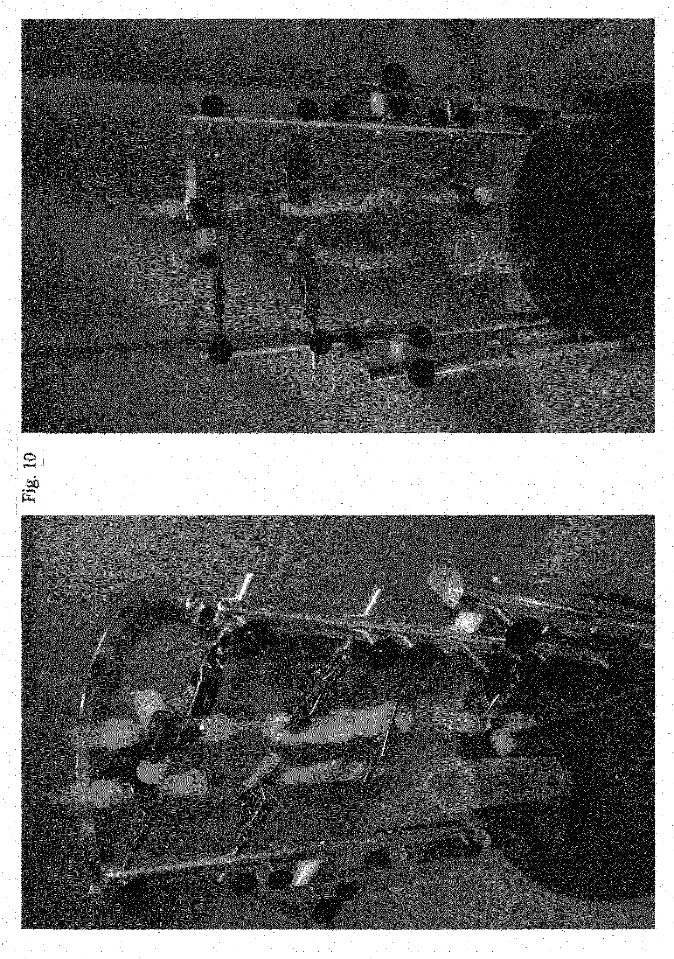

- Fig. 10:: Foto einer Nabelschnurhalterung mit eingespannter, kanülierter Arterie und Vene, mit Durchlauf der Spüllösung

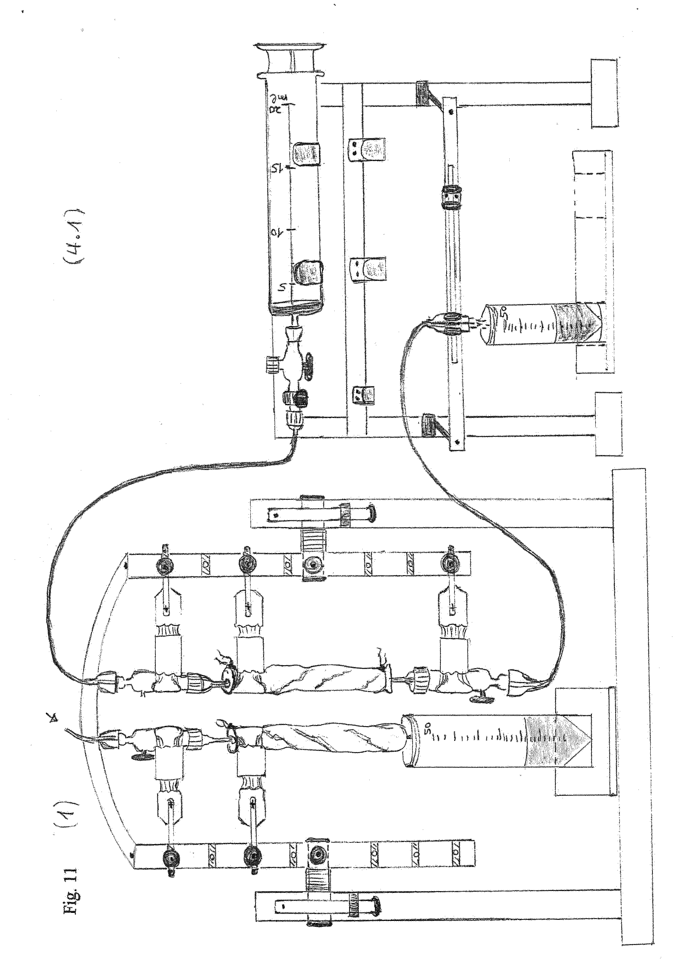

- Fig. 11:: Nabelschnurhalterung mit 2 fixierten, kanülierten Nabelschnurabschnitten (Arterie und Vene) und Spritzenhalterung (Stativ) mit Auffangröhrchen

## Patentansprüche

1. Eine Halterung für die manuelle Isolierung verschiedener Zelltypen aus der Nabelschnur und ein separates Stativ mit Auflageflächen für das einleitende wie ableitende Schlauch-Spritzensystem, ist **dadurch gekennzeichnet, dass**
1.1. das äußere Gestell der Nabelschnurhalterung, bestehend aus
1.1.1 2 senkrechten Metallstäben
1.1.2 oder aus Kunststoff
1.1.3 eine Höhe von 170 - 250 mm
1.1.4 eine Bereite von 8 - 20 mm
1.1.5 eine Bohrung von 0 3 - 10 mm in der Höhe von 80 - 170 mm hat,
1.1.6 1.1.5 zur Frontseite geöffnet ist, für die Entnahme der inneren Halterung (1.4)
1.1.7 auf der Rückseite eine Gewindebohrung von 0 2 - 5 mm im 90° Winkel zu 1.1.5 hat
1.1.8 Flügel- oder Kugelschraube zu 1.1.7
1.1.9 1.1.1/2 im Sockel (1.3) angeschraubt ist
1.1.10 ein Metallriegel, um die innere Halterung (1.4) aus 1.1.6 entnehmen zu können
- B: 2-6 mm
- H: 30 mm
- der 15 mm oberhalb von 1.1.5 mit einer Schraube und Unterlegscheibe angebracht ist
- der 10 - 15 mm unterhalb von 1.1.5 in einen offenen Metallbügel zur Arretierung eingeschwenkt wird, oder
1.1.11 eine Halbschale, um die innere Halterung (1.4) entnehmen zu können
- B: 6 mm
- H: 30 mm
- mit Führungsschienen an 1.1.1 angebracht ist
- oberhalb und unterhalb von 1.1.5
- die Führungsschiene am oberen Rand einen Stoppwulst hat und einen Wulst 20 mm darunter
- 2 Metallbügel an 1.1.1, in denen die Halbschale nach oben und unten geschoben werden kann
- 1. Metallbügel 15 mm oberhalb von 1.1.5
- 2. Metallbügel 10 mm unterhalb von 1.1.5 angebracht ist,
oder
1.1.12 durch Entnahme der Verbindungszapfen zwischen 1.1 und 1.4
1.2 Standsockel zu Anspruch 1.1 ist **dadurch gekennzeichnet, dass**
1.2.1 er aus Kunststoff besteht
1.2.2 rund, 0 210 - 250 mm
1.2.3 oder rechteckig, 100 x 210 - 250 mm sein kann
1.2.4 H: 10 - 20 mm
1.2.5 2 Bohrungen für 1.1. im Abstand von ca. 200 mm hat,
oder
1.3 Standsockel zu Anspruch 1.1, der **dadurch gekennzeichnet ist, dass**
1.3.1 es ein Metallkreis ist
1.3.2 der einen inneren 0 von 185 mm und einen äußeren 0 von 210 mm hat
1.3.3 der in einem Metallkranz steckt
1.3.4 der Metallkreis bei Bedarf im -kranz gedreht werden kann
1.3.5 2 Bohrungen für 1.1 im Abstand von ca. 200 mm vorhanden sind
1.4 die innere Halterung zu Anspruch 1.1 bestehend aus 2 Metallstäben ist **dadurch gekennzeichnet, dass**
1.4.1 sie eine Höhe von 130 - 230 mm
1.4.2 eine Breite von 8 - 15 mm
1.4.3 einen mittigen Drehpunkt mit einem Ø von 3 - 10 mm
1.4.4 sie mehrere Bohrungen für Klemmen (1.5) aufweisen
- von oben nach unten im Abstand von 10 - 20 mm 1.4.5 sie im Abstand von ca. 130 mm zueinander stehen
1.4.6 sie bei Bedarf, aus 1.1.6 entnommen werden können,
- durch Ausschwenken des Metallriegels (1.1.10)
- durch Hochschieben der Halbschalen (1.1.11)
- durch Entnahme der Verbindungszapfen (1.1.12, 1.4.8 / 9)
1.4.7 zu jeder Bohrung von 1.4.4 im 90° Winkel eine Gewindebohrung zur Frontseite vorhanden ist
- 0 von 2 - 5 mm
- in die eine Flügelschraube eingedreht werden kann, um die Klemmen zu arretieren
1.4.8 Nach Anspruch 1.1.5 ein Zapfen die Verbindung zur inneren Halterung (1.4.3) herstellt und **dadurch gekennzeichnet ist, dass**
- er herausnehmbar ist
- 1.4 dadurch ohne Begrenzung schwenkbar ist
- er mit einem überlappenden Schraubenkopf befestigt wird,
oder
1.4.9 eine Rasterschraube zwischen 1.1.5 und 1.4.3 eingesetzt wird
- dadurch 70 - 90 ° nach vorne
- bzw. 110° nach hinten gekippt werden kann
- die mit einem überlappenden Schraubenkopf befestigt wird
1.4.10 1.4.8 oder 1.4.9 läuft durch eine Teflonröhre
- L: 14 - 20 mm; Ø 4 - 12 mm
1.5 Nach Anspruch 1.4.4 können Klemmen in die Bohrungen eingeschoben werden, die **dadurch gekennzeichnet sind, dass**
1.5.1 sie an Führungsstäbe
- angeschraubt oder
- fest verbunden sind
- am äußeren Ende 4-6 Rillen (L: 20 - 30 mm) haben
- Klemme und Führungsstab sind ca. 70 - 10 mm lang
1.5.2 1.5.1 werden in 1.4.4 je nach Bedarf
- eingeschoben
- und an der Frontseite von 1.4 mit einer z. B.
Flügelschraube in 1.4.7 justiert
1.5.3 Klemmfläche von 1.5 kann
- wie eine chirurgische Klemme gearbeitet sein
- die 10 mm hohe Klemmfläche sollte senkrecht zur Nabelschnur stehen
- oder 1.5.3 besteht aus 3 - 5 stumpfen Greifkrallen
- oder 1.5.3 ist wie eine Krokodilklemme
- oder eine Bulldog-Klemme gearbeitet
1.5.4 Klemmkraft von 1.5 ist durch
- Einrasten einer Nase in eine Rille (1.5.3 a)
- eine starke Metallfeder oder
- das Zusammendrücken (Öffnen der Klemme) Lösen der Zangenblätter (Schließen der Klemme) gegeben
1.6 Nach Anspruch 1.4 die Verbindung der inneren Halterung **dadurch gekennzeichnet ist, dass**
1.6.1 oben ein Halbring Ø 130 mm zur Stabilisierung von 1.4
1.6.2 aus Metall oder
1.6.3 aus Kunststoff
1.6.4 aufgesteckt oder
1.6.5 angeschraubt ist
1.6.6 ein zweiter Halbring mit 0 100 mm ausgetauscht werden kann

2. Verfahren nach Anspruch 1.3 ist **dadurch gekennzeichnet, dass** unterhalb der Nabelschnur eine flache Metallschale auf den Sockel der Halterung gestellt wird,
2.1. die rund, 0 120 mm oder
2.2 eckig, 120 x 120 mm sein kann.

3. Verfahren nach Anspruch 1.1 ist **dadurch gekennzeichnet, dass** eine Lupe angebracht ist
3.1 die einschwenkbar
3.2 leicht abnehmbar ist

4. Verfahren nach Anspruch 1 zur Herstellung einer Spritzenhalterung ist **dadurch gekennzeichnet, dass**
4.1 ein Stativ mit Auflagefläche für 2 Spritzen
4.1.1 einen Sockel aus Kunststoff
- H: 5 - 15 mm
- B: 40 mm
- L: 100 mm
4.1.2 2 Metallstangen oder
- 2 Kunststoffstangen
- eckig oder
- rund
- eine H: 195 x B: 8 - 15 mm
- die in 4.1.1 mit einer Schraube oder
- durch ein Gewinde verbunden sind
4.1.3 2 Querstreben
- L: 130 x H: 10 x B: 2 - 3 mm
- in 190 mm und 150 mm Höhe angeschraubt sind
4.1.4 an den beiden Querstreben sind nach Anspruch 4.1.3 Halterungen für die Spritzen angebracht, die **dadurch gekennzeichnet sind, dass**
- eine kleine 0 10 mm
- und zwei große 0 23 - 25 mm
- offene / geschlossene Metallringe oder
- Gummispange(n), daran angebracht sind
4.1.5 1 Querstrebe
- L: 130 x H: 10 x B: 2 - 3 mm
- die mittig einen Schlitz aufweist
- der ca. 80 mm lang und 2 -4 mm hoch ist
- die Querstrebe über einen U-förmigen Steg
- L: 30 - 40 mm; B: 5 mm
- an 4.1.2 in 125 mm Höhe angebracht ist oder
- beidseitig durch ein Rohr an 4.1.2 angeschraubt ist
- L: 30 - 40 mm; 0 3 mm
4.1.6 Verfahren nach Anspruch 4.1.5 ist **dadurch gekennzeichnet, dass**
- 2 Metallspangen oder
- 2 Gummispangen
- an 4.1.5 in dem Schlitz von der Rückseite mit einer Flügelschraube arretiert werden
- von links nach rechts verschoben werden können
4.2 Nach Verfahren 4 zur Herstellung einer Spritzenhalterung kann es ebenso ein Tisch mit einer Auflagefläche für 2 Spritzen sein und ist **dadurch gekennzeichnet, dass**
4.2.1 ein Sockel aus Kunststoff
- H: 5 - 15 x B: 40 x L: 110 mm
4.2.2 4 Säulen
- aus Metall
- oder Kunststoff
- H: 160 0 8 - 12 mm
- sie von der Sockelunterseite mit einer Schraube verbunden sind oder
- durch ein Gewinde in 4.2.1 eingeschraubt sind
4.2.3 die Auflagefläche für Spritzen
- L: 128 x 90 x 5 mm
- aus Metall oder Kunststoff
- 2 Vertiefungen aufweist
- L: 88 x B: 22 mm
- mit einem Seitenabstand von 10 mm
- Zwischenraum zwischen beiden Vertiefungen 26 mm
- 2 - 3 U-förmige Stege
- H: 8 x B: 8 - 20 mm; oberes T-Stück 5-10 mm
- die Stege von unten an die Plattform angeschraubt
- oder angeschweißt sind
4.2.4 2 offene Spangen Ø 10 mm
- aus Metall
- oder Gummi
- die 30 mm vor der Vertiefung für die Spritzen an 4.2.3 angeschraubt sind
4.2.5 Querstrebe an der Längsseite von 4.2 angebracht
- L: 128 x H: 10 mm
- die mittig einen Schlitz aufweist
- der ca. 80 mm lang und 2 -4 mm hoch ist
4.2.6 ist nach Anspruch 4.2.5 **dadurch gekennzeichnet, dass**
- 2 Metallspangen oder
- 2 Gummispangen
- an 4.2.5 in dem Schlitz von der Rückseite mit einer Flügelschraube arretiert werden
- von links nach rechts verschoben werden können

5. Nach Anspruch 1 sind alle Teile, ausgenommen der Einmalartikel, **dadurch gekennzeichnet, dass** sie
5.1 mehrfach sterilisierbar sind
5.2 und Metall nicht rostend ist.

6. Nach Anspruch 1 werden Zusatzteile für die Verarbeitung der Nabelschnur benötigt:
- Klemmen aus nicht rostendem Metall
- Einmalschlauchsystem z.B. Combidyn Druckschlauch Firma Braun Melsungen, GER; REF:520 5000
- Knopfkanülen aus Kunststoff(Einmalartikel)und Metall z. B. Firma Interlock Medizintechnik, Lensahn, GER; Kunststoffeinmalkanülen:
REF: 05053; L: 45 mm, Ø 0,8 mm;
REF: 05050; L: 70 mm, Ø 0,8 mm;
Edelstahlkanülen:
Firma Acufirm, Dreieich; GER REF: 1428LL; L:41mm; Ø 0,8 mm
- Dreiwegehähne
- 20 ml Spritzen
- 50 ml Polypropylen-Röhrchen
- Chirurgischer Faden
z.B. Firma Resorba, Nürnberg, GER;
Supolene 3/0 USP; REF: 91513;
- Bulldog-Klemme
z. B. Fa. Ulrich medical Ihox
Sensi-Mikro-Chip; 34 mm; UL 4150-04
oder Bulldog-Klemme nach Potts; Feder verstellbar 40 mm; UL 4170-04
- Skalpell
- Sterile Mullkompressen
- Frischhaltefolie
- Wärme-Gelkompresse (37°C)
- Lupe mit Schwenkarm, die evtl. am Gestell 1.1 oder 1.4 befestigt werden kann, bzw. freistehend.

## Geänderte Patentansprüche

### Geänderte Patentansprüche gemäss Regel 137(2) EPÜ.

1. Eine Halterung für die manuelle Isolierung verschiedener Zelltypen aus der Nabelschnur und ein separates Stativ mit Auflageflächen für das einleitende wie ableitende Schlauch-Spritzen-System, sowie eine Wärme-Inkubationseinheit, ist **dadurch gekennzeichnet, dass**
1.1 das äußere Gestell der Nabelschnurhalterung, bestehend aus
1.1.1 1 - 2 senkrechten Metallstäben
1.1.2 oder aus Kunststoff
1.1.3 H: 150 - 250 mm
1.1.4 B: 8 - 20 mm
1.1.5 einer Bohrung etwa 10 -20 mm (a) und 70 - 80 mm (b) vom oberen Ende und 20 mm (c) vom unteren Ende,
- Ø: 3 - 10 mm
- evtl. über ein Gewinde verfügen
1.1.6 1.1.5 zur Frontseite geöffnet ist, für die Entnahme der inneren Halterung (1.4)
1.1.7 auf der Rückseite eine Gewindebohrung von
- Ø 2 - 5 mm
- im 90° Winkel zu 1.1.5 hat
1.1.8 Flügel- oder Kugelschraube zu 1.1.7
1.1.9 1.1.1/2 im Sockel (1.3) angeschraubt ist
1.1.10 ein Metallriegel, um die innere Halterung (1.4) aus 1.1.6 entnehmen zu können
- B: 2-6 mm
- H: 30 mm
- der 15 mm oberhalb von 1.1.5.a mit einer Schraube und Unterlegscheibe angebracht ist
- der 10 - 15 mm unterhalb von 1.1.5.a in einen offenen Metallbügel zur Arretierung eingeschwenkt wird, oder
1.1.11 eine Halbschale, um die innere Halterung (1.4) entnehmen zu können
- B: 6 mm
- H: 30 mm
- mit Führungsschienen an 1.1.1 angebracht ist
- oberhalb und unterhalb von 1.1.5
- die Führungsschiene am oberen Rand einen Stoppwulst hat und einen Wulst 20 mm darunter
- 2 Metallbügel an 1.1.1, in denen die Halbschale nach oben und unten geschoben werden kann
- 1. Metallbügel 15 mm oberhalb von 1.1.5
- 2. Metallbügel 10 mm unterhalb von 1.1.5 angebracht ist,
oder
1.1.12 durch Entnahme der Verbindungszapfen zwischen 1.1 und 1.4
oder
1.1.13 nach Anspruch 1.1.1. der Metallstab in die Hülse (1.3.5) eingesetzt und mit einem Stift darin justiert (Fig. 13) wird (1.1.5.c)
1.1.14 2 offenen Spangen die am Metallstab (1.1.1) angebracht sind, in die während der Wärmeinkubation die Enden des Schlauchsystems eingeklickt werden, damit nach Absenken der Kunststoffglocke ein geschlossenes System besteht
1.2 Standsockel zu Anspruch 1.1 ist **dadurch gekennzeichnet, dass**
1.2.1 er aus Kunststoff besteht
1.2.2 rund, Ø 210 - 250 mm
1.2.3 oder rechteckig, 100 x 210 - 250 mm sein kann
1.2.4 H: 10 - 20 mm
1.2.5 2 Bohrungen für 1.1 im Abstand von ca. 100 - 200 mm hat, oder
1.3 ein Standsockel zu Anspruch 1.1, der **dadurch gekennzeichnet ist, dass**
1.3.1 es ein Metallkreis ist
1.3.2 der einen inneren (a) Ø von 185 mm und einen äußeren (b) Kreis Ø von 210 mm hat, evtl. noch einen mittleren Ring (c) von etwa 195 mm aufweist
1.3.3 die zwei bzw. drei Metallkreise a + b sind durch mehrere senkrecht stehende Streben, L: 20 - 30 mm, miteinander verbunden, dadurch ergibt sich eine abgeschrägte standfeste Basisstation (Fig. 15)
1.3.4 auf 1.3.2.a sind diagonal 1 - 2 Metallschienen (Fig. 16) angebracht
- sie können flach oder
- rund gearbeitet sein
1.3.5 auf den Metallschienen sitzen 1 - 3 offene Hülsen
- aus Metall
- Innen Ø: 9 - 13 mm
- H: 30 - 40 mm
- im Abstand von 40 -100 mm
1.3.6 sie haben in 20 mm Höhe 4 diagonal gegenüber liegende Bohrungen,
- Ø: 3 - 6 mm
- ein Metall- oder Kunststoffstift Ø: 2 - 5 mm, L: 15 - 20 mm,
- mit verdicktem Kopfende
- oder in Form eines Imbusschlüssels
- wird durch 1.3.5 geschoben und in 1.1.5.c (Fig. 13) gesteckt oder geschraubt, wodurch ein Umkippen der Halterung (1.4) ausgeschlossen ist
1.3.7 auf 1.3.2.a links und rechts zwei 40 - 50 mm hohe Stäbe sitzen
- Ø: 5 - 10 mm
1.4 die innere Halterung zu Anspruch 1.1 bestehend aus einem Metallstab ist **dadurch gekennzeichnet, dass**
1.4.1 er eine Höhe von 130 - 230 - 35 mm hat, es werden verschiedene Längen bereit gestellt, für die unterschiedlichen Anwendungen
1.4.2 eine Breite von 8 - 15 mm
1.4.3 einen mittigen oder
- exzentrischen Drehpunkt mit einem
- Ø von 3 - 10 mm besitzt
1.4.4 sie mehrere Bohrungen für Klemmen (1.5) aufweisen
- über die gesamte Länge im Abstand von 10 - 20 mm,
- Ø von 3 - 10 mm
1.4.5 sie im Abstand von ca. 100 - 130 mm zueinander stehen
1.4.6 sie bei Bedarf, aus 1.1.5.c entnommen werden können 1.4.7 zu jeder Bohrung von 1.4.4 im 90° Winkel eine Gewindebohrung zur Frontseite vorhanden ist
- Ø von 2 - 5 mm
- in die eine Flügelschraube eingedreht werden kann, um die Klemmenstifte zu arretieren
1.4.8 Nach Anspruch 1.1.5.a ein Zapfen die Verbindung zur inneren Halterung (1.4.3) herstellt und **dadurch gekennzeichnet ist, dass**
- er herausnehmbar ist
- 1.4 dadurch ohne Begrenzung schwenkbar ist
- er mit einem überlappenden Schraubenkopf befestigt wird,
oder
1.4.9 eine Rasterschraube zwischen 1.1.5.a und 1.4.3 eingesetzt wird
- dadurch 70 - 90° nach vorne
- bzw. 110° nach hinten gekippt werden kann
- die mit einem überlappenden Schraubenkopf befestigt wird
1.4.10 1.4.8 oder 1.4.9 läuft durch eine Teflonröhre, um den Abstand zu 1.1. zu gewährleisten
- L: 14 - 30 mm
- Ø 4 - 12 mm
1.4.11 ein Stift z.B. in Form eines Imbussschlüssels von 1.1.5.b) nach 1.4 durchgeschoben wird, um die innere Halterung zu arretieren (Fig. 13)
1.5 Nach Anspruch 1.4.4 können Klemmen in die Bohrungen eingeschoben werden, die **dadurch gekennzeichnet sind, dass**
1.5.1 sie an Führungsstäbe
- abgeschraubt oder
- fest verbunden sind
- am äußeren Ende 4 - 6 Rillen (L: 20 - 30 mm) haben
- Klemme und Führungsstab sind ca. 70 - 100 mm lang
1.5.2 1.5.1 werden in 1.4.4 je nach Bedarf
- eingeschoben
- und an der Frontseite von 1.4 mit einer z. B. Flügelschraube in 1.4.7 justiert
1.5.3 Klemmfläche von 1.5 kann
- wie eine chirurgische Klemme gearbeitet sein
- die 5 - 10 mm hohe Klemmfläche sollte senkrecht zur Nabelschnur stehen
- oder 1.5.3 besteht aus 3 - 5 stumpfen Greifkrallen
- oder 1.5.3 ist wie eine Krokodilklemme
- oder eine Bulldog-Klemme gearbeitet
1.5.4 Klemmkraft von 1.5 ist durch
- Einrasten einer Nase in eine Rille (1.5.3. a)
- eine starke Metallfeder oder
- das Zusammendrücken (Öffnen der Klemme) Lösen der Zangenblätter (Schließen der Klemme) gegeben
1.6 Nach Anspruch 1.4 die Verbindung der inneren Halterung je nach Ausführung **dadurch gekennzeichnet ist, dass**
1.6.1 oben ein Halbring Ø 130 mm zur Stabilisierung von 1.4
1.6.2 aus Metall oder
1.6.3 aus Kunststoff
1.6.4 aufgesteckt oder
1.6.5 angeschraubt ist
1.6.6 ein zweiter Halbring mit Ø 100 mm ausgetauscht werden kann

2. Verfahren nach Anspruch 1.3 ist **dadurch gekennzeichnet, dass** unterhalb der Nabelschnur eine flache Metallschale in den Freiraum des Metallkranzes gestellt wird,
2.1. die rund, Ø 70 - 90 mm oder
2.2 eckig, 100 x 80 mm sein kann. Sterilraum für die Zellablösung bilden

3. Verfahren nach Anspruch 1. ermöglicht die Wärme-Inkubationseinheit die Inkubation des Gefäßsegmentes mit einer Enzymlösung bei einer definierten Temperatur von z.B. 37°C plus Befeuchtung unter der Laminar Air Flow und ist **dadurch gekennzeichnet, dass** sie aus dem Heizelement, einer Adapterplatte mit umlaufender Rinne, einer Wasserschale und einer Glocke besteht und zusammen einen abgeschlossenen Sterilraum für die Zellablösung bilden
3.1 ein Heizelement, dass **dadurch gekennzeichnet ist, dass** es eine
3.1.1 Elektroeinheit besitzt
- 240 x 240 mm
- Display mit
- Temperaturanzeige
- Taste: T ▼
- Taste: T ▲
- Menütaste: speichern der fixen Temperatur, Gradienteneingabe
- 2 Buchsen für Temperaturfühler und Feuchtigkeitsmesser
- die Elektroeinheit mit einer durchsichtigen Kunststoffhaube überzogen sein kann, die desinfiziert werden kann
3.1.2 eine Wärmeplatte, auf der Elektroeinheit aufgesetzt ist
- Ø: 150 - 200 mm
3.2 Nach Anspruch 3 eine Adapterplatte, die **dadurch gekennzeichnet ist, dass** sie
- wärmeleitend
- aus Metall
- die Größe auf den Durchmesser der Wärmeplatte abgestimmt ist
- Innen Ø 150 - 170 mm
- Außen Ø 170 - 230 mm
3.2.1 eine Rinne den Bonden der Adaperterplatte umgibt (Fig. 13)
- B: 20- 30 mm
- H: 20 mm über den Plattenboden, 10 - 20 mm unter den Plattenboden
- die Rinne besitzt umlaufend mittig einen Steg von 5 - 10 mm, in der (1.3) eingestellt und darüber im äußeren Rund die Glockenhaube (3.3) abgesenkt wird
- sterilisierbar
3.3 Nach Anspruch 3 eine Kunststoffglocke, Glas-, Metall-, die **dadurch gekennzeichnet ist, dass** sie
3.3.1 aus Polypropylen oder ähnlichem besteht
- Dampf sterilisierbar 124°C
- unzerbrechlich ist
- H: 250 - 350 mm
- B: 210 - 250 mm
3.3.2 2 - 3 Öffnungen, die in der Kuppel bzw. im oberen Drittel der Glocke plaziert sind, für:
a. Temperaturfühler
b. Feuchtigkeitsmesser (% Luftfeuchtigkeit)
c. Belüftungsventil
d. bei Nichtbedarf werden die Öffnungen mit einem Blindverschluß gesichert
e. die Öffnungen (3.3.2.c) haben eine verstärkte Führungsröhre, die eingeschraubt und ebenso separat gesäubert und sterilisiert wird
3.3.3 die Kuppel besitzt 3 - 4 Standbeine, damit sie umgedreht Platz sparend beiseite gestellt werden kann und der Innenraum steril bleibt
3.4 eine Metallschale mit 20 - 50 ml sterilem Aqua destillata

4. Verfahren nach Anspruch 1 zur Herstellung einer Spritzenhalterung ist **dadurch gekennzeichnet, dass**
4.1 ein Stativ mit Auflagefläche für 2 Spritzen
4.1.1 einen Sockel aus Kunststoff
- H: 5 - 15 mm
- B: 40 mm
- L: 100 mm
4.1.2 2 Metallstangen oder
- 2 Kunststoffstangen
- eckig oder
- rund
- eine H: 195 x B: 8 - 15 mm
- die in 4.1.1 mit einer Schraube oder
- durch ein Gewinde verbunden sind
4.1.3 2 Querstreben
- L: 130 x H: 10 x B: 2 - 3 mm
- in 190 mm und 150 mm Höhe angeschraubt sind
4.1.4 an den beiden Querstreben sind nach Anspruch 4.1.3 Halterungen für die Spritzen angebracht, die **dadurch gekennzeichnet sind, dass**
- eine kleine Ø 10 mm
- und zwei große Ø 23 - 25 mm
- offene / geschlossene Metallringe oder
- Gummispange(n), daran angebracht sind
4.1.5 1 Querstrebe
- L: 130 x H: 10 x B: 2 - 3 mm
- die mittig einen Schlitz aufweist
- der ca. 80 mm lang und 2 -4 mm hoch ist
- die Querstrebe über einen U-förmigen Steg
- L: 30 - 40 mm; B: 5 mm
- an 4.1.2 in 125 mm Höhe angebracht ist oder
- beidseitig durch ein Rohr an 4.1.2 angeschraubt ist
- L: 30 - 40 mm; Ø 3 mm
4.1.6 Verfahren nach Anspruch 4.1.5 ist **dadurch gekennzeichnet, dass**
- 2 Metallspangen oder
- 2 Gummispangen
- an 4.1.5 in dem Schlitz von der Rückseite mit einer Flügelschraube arretiert werden
- von links nach rechts verschoben werden können
4.2 Nach Verfahren 4 zur Herstellung einer Spritzenhalterung kann es ebenso ein Tisch mit einer Auflagefläche für 2 Spritzen sein und ist **dadurch gekennzeichnet, dass**
4.2.1 es ein Sockel aus Kunststoff
- H: 5 - 15 x B: 40 x L: 110 mm
4.2.2 4 Säulen
- aus Metall
- oder Kunststoff
- H: 160 Ø 8 - 12 mm
- sie von der Sockelunterseite mit einer Schraube verbunden sind oder
- durch ein Gewinde in 4.2.1 eingeschraubt sind
4.2.3 die Auflagefläche für Spritzen
- L: 128 x 90 x 5 mm
- aus Metall oder Kunststoff
- 2 Vertiefungen aufweist
- L: 88 x B: 22 mm
- mit einem Seitenabstand von 10 mm
- Zwischenraum zwischen beiden Vertiefungen 26 mm
- 2 - 3 U-förmige Stege
- H: 8 x B: 8 - 20 mm; oberes T-Stück 5-10 mm
- die Stege von unten an die Plattform angeschraubt
- oder angeschweißt sind
4.2.4 2 offene Spangen Ø 10 mm
- aus Metall
- oder Gummi
- die 30 mm vor der Vertiefung für die Spritzen an 4.2.3 angeschraubt sind
4.2.5 Querstrebe an der Längsseite von 4.2 angebracht
- L: 128 x H: 10 mm
- die mittig einen Schlitz aufweist
- der ca. 80 mm lang und 2 -4 mm hoch ist
4.2.6 ist nach Anspruch 4.2.5 **dadurch gekennzeichnet, dass**
- 2 Metallspangen oder
- 2 Gummispangen
- an 4.2.5 in dem Schlitz von der Rückseite mit einer Flügelschraube arretiert werden
- von links nach rechts verschoben werden können

5. Nach Anspruch 1 sind alle Teile, ausgenommen der Einmalartikel, **dadurch gekennzeichnet, dass** sie
5.1 mehrfach sterilisierbar sind
5.2 und Metall nicht rostend ist.

6. Nach Anspruch 1 werden Zusatzteile für die Verarbeitung der Nabelschnur benötigt:
- Klemmen aus nicht rostendem Metall
- Einmalschlauchsystem z.B. Combidyn Druckschlauch Firma Braun Melsungen, GER; REF:520 5000
- Knopfkanülen aus Kunststoff(Einmalartikel)und Metall z. B. Firma Interlock Medizintechnik, Lensahn, GER; Kunststoffeinmalkanülen:
REF: 05053; L: 45 mm, Ø 0,8 mm;
REF: 05050; L: 70 mm, Ø 0,8 mm;
Edelstahlkanülen:
Firma Acufirm, Dreieich; GER REF: 1428LL; L:41mm; Ø 0,8 mm
- Dreiwegehähne
- 20 ml Spritzen
- 50 ml Polypropylen-Röhrchen
- Chirurgischer Faden
z.B. Firma Resorba, Nürnberg, GER;
Supolene 3/0 USP; REF: 91513;
- Bulldog-Klemme
z. B. Fa. Ulrich medical Ihox
Sensi-Mikro-Chip; 34 mm; UL 4150-04
oder Bulldog-Klemme nach Potts; Feder verstellbar 40 mm; UL 4170-04
- Skalpell
- Lupe mit Schwenkarm, die evtl. am Gestell 1.1 oder 1.4 befestigt werden kann, bzw. freistehend
